# EUROPEAN PATENT SPECIFICATION

(11) **EP 0 941 229 B1**
(45) Date of publication and mention of the grant of the patent: **03.09.2003**
(21) Application number: 97951975.8
(22) Date of filing: 25.11.1997
(51) Int. Cl.: C07D 503/02

(54) **PURIFICATION OF FERMENTED CLAVULANIC ACID**
REINIGUNG VON FERMENTIERTER CLAVULANSÄURE
PURIFICATION D'ACIDE CLAVULANIQUE FERMENTE

(30) Priority: 27.11.1996 AT 206296
(43) Date of publication of application: 15.09.1999
(73) Proprietor: BIOCHEMIE Gesellschaft m.b.H., 6250 Kundl Tirol (AT)
(72) Inventor: SUMMER, Harald, A-6300 Woergl (AT); RENZL, Alois, A-6233 Kramsach 208 (AT); WAGNER, Helmut, A-6233 Kramsach (AT)
(74) Representative: Grubb, Philip William
(86) International application number: EP9706578
(87) International publication number: WO98023622

(56) References cited:
- EP-A- 0 026 044
- EP-A- 0 385 552
- EP-A- 0 387 178
- EP-A- 0 594 099
- WO-A-93/25557
- WO-A-94/21647
- WO-A-95/23870
- WO-A-95/34194
- WO-A-96/20199
- WO-A-96/28452
- WO-A-96/33197
- GB-A- 1 508 977

## Description

The present invention relates to a process for the purification of fermented products, *i.e*. in the production of pharmaceutically acceptable salts of clavulanic acid of formula 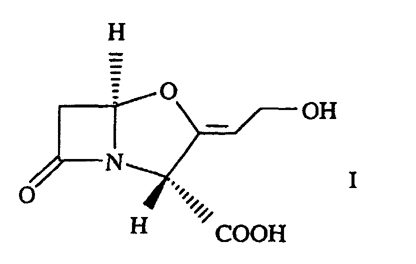 *e.g*. potassium clavulanate.

Pharmaceutically acceptable salts of clavulanic acid are known and various production processes, for example *via*
a) fermentation of a micro-organism which is capable to produce clavulanic acid
b) isolation of clavulanic acid from the fermentation broth
c) purification of clavulanic acid, *e.g. via* a salt thereof
d) conversion of the purified clavulanic acid of step c), *e.g.* of a salt thereof into a pharmaceutically acceptable salt of clavulanic acid
are disclosed.
EP-A-0385 552 discloses a process for the purification of fermentation broths obtained from fermentations with *Streptomyces* strains by adding of an inorganic electrolyte together with an inorganic coagulation agent at a pH of 5-8, then adding an organic flocculation agent in order to separate finally the flocculi. The resulting transparent liquid may undergo further process steps to isolate the product, e.g. clavulanic acid.
EP-A-0-387 178 concerns a process for the production of clavulanic acid or a salt thereof including the steps of flocculation of the fermentetion broth using an inorganic electrolyte as a coagulation agent along with an organic polyelectrolyte, decanting the flocculi and purification of clavulanic acid by forming an amine salt of clavulanic acid, isolating it and converting it into a pharmaceutically acceptable salt, e.g. the potassium salt of clavulanic acid.
WO95/23870 relates to a process for the isolation of clavulanic acid or a pharmaceutically acceptable salt thereof wherein the cells, a major part of proteins and other suspended solid particles are removed by microfiltration at a pH of 5.8 to 6.2, the purified aqueous phase is optionally additionally purified by ultrafiltration and further concentrated by reverse osmosis. In the next step, the clavulanic acid is extracted into a water-immiscible, organic solvent at a pH value of pH 1 to 3 which is dried, decolourized and concentrated, e.g. by evaporation. Then, the clavulanic acid present in the organic phase is isolated and purified by forming a salt with an amine, in particular N,N'-diisopropylethylenediamine, which is isolated and converted into a pharmaceutically acceptable potassium salt of clavulanic acid such as the potassium salt that is again isolated.
WO96/33197 concerns an improvement of the process disclosed in WO95/23870 by adding an additional solvent, e.g. an alcohol, to a solution of clavulanic acid in a water-immiscible solvent obtained from an extraction of the pre-concentrated and pre-purified aqueous fermentation broth resulting in high yields and purities of clavulanic acid or a pharmaceutically acceptable salt or ester thereof.

It has now been found that the production of a pharmaceutically acceptable salt of clavulanic acid, *e.g.* such as the potassium salt may surprisingly be improved, if the aqueous fermentation broth is extracted with a solvent before clavulanic acid is isolated. Due to such an extraction prior to isolation, clavulanic acid, *e.g*. in form of a pharmaceutically acceptable salt, may be obtained in surprising pure form and surprising high yields from the fermentation broth.

In one aspect the present invention provides a process for the production of clavulanic acid and pharmaceutically acceptable salts thereof by
a) fermentation of a micro-organism which is capable to produce clavulanic add to provide a fermentation broth containing clavulanic acid
b) extraction of the fermentation broth containing clavulanic add with an organic solvent that is able to form a liquid/liquid phase system in contact with an aqueous fermentation broth, selected from the group consisting of an ether, a ketone, an ester, an alcohol and a mixture thereof at a pH of 5.5 to 7.5 to provide an extracted aqueous fermentation broth whereby the clavulanic acid remains substantially in the aqueous fermentation broth,
c) extraction of clavulanic acid from said extracted aqueous fermentation broth at a pH of 1.5 to 2.5 with a solvent in which clavulanic acid is soluble under the extraction conditions and which is able to form a liquid/liquid phase system in contact with the aqueous fermentation broth, and which is selected from the group consisting of an ester, a ketone, an alcoholand a mixture thereof,
d) purification of the extracted clavulanic acid obtained in step c) ,or a salt thereof, and
e) conversion of the purified clavulanic acid of step d), or a salt thereof into a pharmaceutically acceptable salt of clavulanic acid, *e.g*. potassium clavulanate.

Fermentation step a) may be carried out as usual, for example, as generally described *e.g*. in GB 1508977 and WO 93/25557. A particular fermentation process, is disclosed, *e.g*. in EP 182 522 by continuously or intermittently feeding a carbon source during fermentation; *e.g*. in WO 96/18743 by keeping low levels of ammonium and urea; *e.g.* in WO 97/19187 by strict control of the soluble phosphate content in the fermentation medium; *e.g*. in EP 349 121; WO 95/03416; CA 2108113; WO 94/18326; WO 94/12654; and WO 96/10084; by production of clavulanic acid from a host transformed with a vector comprising a DNA or a DNA fragment that is encoding at least one enzyme involved in clavulanic acid production. An appropriate micro-organism may be for example a micro-organism of the genus *Streptomyces,* such as *S. clavuligerus*, *i.e.* strain NRRL 3585, or *Streptomyces sp.* P6621 FERM 2804 (Japanese patent 55,162,993) or other mutants. Fermentation under appropriate conditions is in more detail known from various publications, *e.g*. from references cited herein under step a) und under steps c), d) and e) below, the content of which, including prior art citations therein, is incorporated herein by reference.

According to the present invention the fermentation broth is extracted in step b) prior to isolation of clavulanic acid. Extraction of an aqueous fermentation broth in step b) may *e.g*. be carried out as follows:
Extraction may be carried out as usual, e.g. according to any method disclosed in any of the references cited under step a) above, or step c), d) and e) below.
The entire unfiltered fermentation broth,
or, a fermentation broth from which at least part of the suspended solids have been removed, for example by flocculation, *e.g*. by pH adjustment or addition of flocculation agent(s); by filtration, *e.g*. by precoat filtration on a rotary vacuum filter, *e.g*. assisted by a filtering aid, for example based on mineral or cellulose; by crossflow filtration with or without prior sieving of coarse particles; by microfiltration; or by centrifugation
may be extracted with a solvent, selected from the group consisting of an ether, a ketone, an ester, an alcohol and a mixture thereof, which is able to form a liquid/liquid phase system in contact with an aqueous fermentation broth, *e.g.* which is immiscible with the broth.
The aqueous, clavulanic acid containing liquid may be pre-concentrated prior to extraction, to achieve specific concentration ranges, *e.g*. by anion exchange or osmotic methods to a concentration of clavulanic acid of 20 to 80 g/kg, such as 10 to 50 g/kg, *e.g*. 10 to 40 g/kg, for example 10 to 30 g/kg.

In another aspect the present invention provides a process as defined above, characterized in that at least part of the solids suspended in the fermentation broth are removed before extraction; and a process as defined above, characterized in that the fermentation broth is concentrated prior to extraction.

A solvent for extraction, which is able to form a liquid/liquid phase system in contact with an aqueous fermentation broth, includes ethers, ketones, *e.g.* methyl isobutyl ketone, diethylketone, methylethyl ketone; esters, *e.g*. ethyl acetate, a propylacetate; or alcohols, such as a butanol, for example n-butanol or iso-butanol, preferably ethyl acetate, diethylketone, methyl isobutylketone, such as ethyl acetate; wherein clavulanic acid should be preferably not or only to a small extent soluble under the extraction conditions, *e.g.* depending on the pH of the fermentation broth. Mixtures of individual solvents, e.g. as described above may be used.
The ratio, *e.g*. volume ration, of the solvent and the fermentation broth is not critical; *e.g*. a ratio of 0.5:1 to 3:1 may be used. One or several extractions may be carried out. An amount of solvent may be used which is sufficient that two liquid phases in contact with the fermentation broth are formed.
Extraction may be carried out at and around a native pH, *e.g.* at a pH of 5.5 to 7.5*.*
Phase separation of the aqueous and the organic phase may be facilitated, for example by centrifugation methods, *e.g*. centrifugal separators or centrifugal decanters.
Removal of impurities, such as dyes or other, *e.g.* biomass residues, of the clavulanic acid containing liquid may be carried out, either prior or subsequent to extraction, *e.g*. as usual, *e.g*. by adsorption, *e.g*. by use of activated carbon in powder form, *e.g*. in a quantity of ca. 0.5% to 5% (based on the weight of a clavulanic acid containing liquid); or by use of activated carbon in granulated form, which when filled into a column may purify an, *e.g.* preconcentrated clavulanic acid containing liquid, percolating through; or by use of adsorber or ion exchange resins, which, *e.g*. may be suitable for selectively removing dyes and other impurities from solvents, *e.g*. by use of such resins in a batch process or column-percolation process; or *e.g.* by other methods, such as a crossflow method, *e.g*. permeation of the clavulanic acid containing liquid through, *e.g*. a membrane of *e.g.* small pore size, such as a pore size of *e.g*. 1 to 3 kD.

Step c) may be carried out *via* extraction from an aqueous solution of clavulanic acid, for example, by adjusting the pH of a fermentation broth to an pH of about 1.5 to 2.5, such as 1.5 to 2.5, at which clavulanic acid is present in free acid form; after the extraction according to the present invention; and
extraction of an pH adjusted, *e.g.* acidified fermentation broth with a solvent in which clavulanic acid is soluble under the extraction conditions, and which is able to form a liquid/liquid phase system in contact with an aqueous fermentation broth, selected from the group consisting of an ester, a ketone, an alcohol and a mixture thereof, *e.g*. by
direct extraction of an *e.g*. acidified fermentation broth, or after removing at least part of the solids suspended in an *e.g*. acidified fermentation broth, *e.g.* according to an extraction method as usual, *e.g*. as described above. Solids may be removed, for example by flocculation, filtration, *e.g.* by microfiltration, or by centrifugation.
A water-miscible solvent may be added to a fermentation broth in order to improve filterability of the fermentation broth prior to solid removal.
An extraction solvent selected from the group consisting of an ester, a ketone, an alcohol and a mixture thereof includes a solvent wherein clavulanic acid is soluble under the extraction conditions, *e.g* ketones, such as methyl-iso-butyl ketone, diethylketone; esters, such as ethyl acetate, a propyl or a butyl acetate or alcohols, such as a butanol, for example n-butanol or iso-butanol, preferably ethyl acetate. Mixtures of individual solvents, *e.g.* as described above may be used. Preferably the same solvent may be used which was used in step b) for extraction of the fermentation broth prior to isolation of clavulanic acid *(e.g.* at or around a native pH of 5.5-7.5). An amount of solvent may be used which is sufficient that two liquid phases in contact with the fermentation broth are formed. Per part of the fermentation broth, *e.g.* 1:1 to 5:1 parts of the solvent may be used.
One or several extractions of the *e.g.* acidified fermentation broth may be carried out.

In another aspect the present invention provides a process as defined above, characterized in that as a solvent for the extraction of clavulanic acid from an aqueous solution thereof the same solvent is used which was used in step b) for extraction of the fermentation broth prior to isolation of clavulanic acid thereof.

An aqueous, clavulanic acid containing liquid may be pre-concentrated, conveniently prior to acidification and clavulanic acid extraction, to achieve specific clavulanic acid concentration ranges by a method as usual, such as by fine vacuum evaporation; or an osmotic method such as crossflow reversed osmosis, crossflow nanofiltration, anion exchange, to obtain a concentration of *e.g*. 20 g to 80 g clavulanic acid per kg in the concentrate, such as 10 to 50 g/kg, *e.g*. 10 to 40 g/kg, for example 10 to 30 g/kg.

Washing of the clavulanic acid containing solvent with water or an appropriate buffer may be carried out, *e.g*. after extraction of the aqueous, clavulanic acid containing liquid. The amount of water or buffer is not critical; *e.g.* conveniently 5% to 100% v/v, such as 5% to 30% v/v water or buffer in respect with the organic extract may be used.
Phase separation of the aqueous and the organic phase may be facilitated, for example by centrifugation methods, e.g. centrifugal separators or centrifugal decanters.
The solution of clavulanic acid in an organic solvent, for example obtainable by extraction, may be *e.g*. back-extracted into water, *e.g.* for further purification.
The solution of clavulanic acid in an *e.g*. organic solvent may be dried to achieve specific water ranges prior to further processing and/or it may be concentrated, *e.g*. as usual, for example by evaporation, *e.g*. by a heteroazeotrope distillation method, or, *e.g.* by solvent resistant membranes {*e.g*. SELRO (R)® or CARBOSEP (R)®] *via* nanofiltration or reversed osmosis, to obtain a desired concentration of clavulanic acid, *e.g*. a concentration of about 20 g to 80 g, *e.g*. 10 g to 40 g clavulanic acid per kg in the *e.g.* organic solvent, *e.g*. prior to purification step d) which is described below.

In another aspect the present invention provides a process as defined above, characterized in that a clavulanic acid containing solvent is concentrated prior to purification step d).

Removal of dyes or other impurities, *e.g.* biomass residues, of the clavulanic acid containing liquid, e.g. organic solvent, may be carried out, *e.g.* as usual, *e.g*. according to a method as described above.
Isolation processes using appropriate conditions are in more detail known from various publications, *e.g.* from EP 387 178; WO 93/25557; WO 95/11295; WO 95/34194; WO 96/28452; WO 96/22296. The content of references cited under step a) and c), and under steps d) and e) below including prior art citations therein, is incorporated herein by reference.

Purification stepd) may for example be carried out by chromatography or *via* salt formation, for example *via* formation of a salt of clavulanic acid that may precipitate, for example crystallise, from the solvent used. Such a salt may be, for example the sodium salt or the lithium salt of clavulanic acid, *e.g.* as described in GB 1543563, or GB 1508977, or an amine salt. If purification is carried out via salt, *e.g.* amine salt formation, a further solvent for clavulanic acid, such as *e.g*. acetone, methyl isobutyl ketone or diethyl ketone, preferably acetone, may be added to the clavulanic acid containing, *e.g*. preconcentrated liquid, *e.g*. in an amount from 0 to 200% based on the weight of the clavulanic acid containing, *e.g.* preconcentrated liquid. Suitable amines which form a salt with clavulanic acid are described in various publications, such as tert.butylamine in EP 26 044; N,N-(di)alkyl-alkylene-diamines, such as diisopropyl-ethylen-diamine in EP 562 583; N,N,N',N'-tetramethyl-ethylendiamine in EP 719 778; tert.octylamine for example in GB 2264944 or EP 594 099; or for example a class of amines in WO 93/25557, wherein are described amines of formula 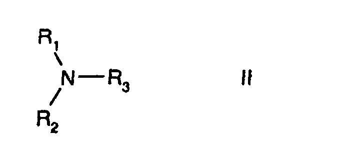 wherein R₁, R₂ and R₃ are selected according to the following options:
da) R₁ being an optionally substituted cyclic group of general formula

   R-(CHR₄)ₘ-

   wherein
   m is zero or an integer 1 to 5;
   R is an optionally substituted aliphatic hydrocarbon ring system containing from 3 to 8 ring carbon atoms;
   R₄ is hydrogen or alkyl, amino- or hydroxy substituted alkyl; or substituted amino-substituted alkyl; or a group of the same general formula R₁ above;
   R₂ and R₃ are independently selected from the same groups from which R₁ is selected; or from hydrogen; alkyl; alkenyl; amino- or hydroxy-substituted alkyl or alkenyl; or substituted amino-substituted alkyl or alkenyl;
   or
db)R_{1,} R₂ and R₃ are the same or different and are independently selected from hydrogen; alkyl; alkenyl; amino- or hydroxy- or alkoxy- substituted alkyl or alkenyl; or substituted amino-substituted alkyl or alkenyl; or
dc) R₁ being an optionally substituted aryl group of general formula 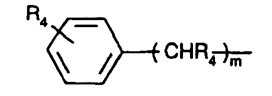 wherein
   R₄ is hydrogen or one or more substituents, and m is zero or an integer of 1 to 5
   R₂ and R₃ are independently selected from hydrogen; alkyl; amino- or hydroxy-substituted alkyl; or substituted amino-substituted alkyl; or groups of the same general formula from which R₁ is selected; or
dd)R₁ and R₂ and optionally R₃ together with the nitrogen atom shown being the residue of an optionally substituted heterocyclic ring system including the nitrogen atom as a ring member, and optionally including one or more additional ring hetero atoms; and, if R₃ is not part of the ring system R₃ is independently selected from hydrogen, alkyl, amino- or hydroxy-substituted alkyl or substituted amino-substituted alkyl; or
de) R₁ being a group of general formula 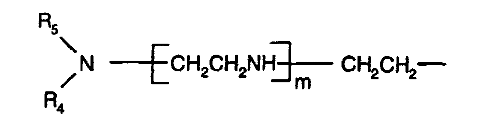 wherein
   R₄ and R₅ are independently hydrogen; alkyl; amino- substituted alkyl; or substituted amino-substituted alkyl; and
   R₂ and R₃ are independently selected from hydrogen; alkyl amino- or hydroxy substituted alkyl; or substituted amino-substituted alkyl; and
   m is zero or an integer of 1 to 5; or
df) one or both of R₁ and R₂ are hydrogen and
   R₃ represents the residue of an amino acid in which the carboxylate group of the amino acid may be esterified or in the form of an amine.

The amine may *e.g*. further be as described in WO 94/22873, such as an amine of formula 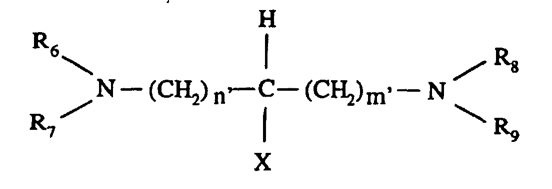 wherein
R₆ and R₇ are each C₁₋₈alkyl; C₃₋₈cycloalkyl; or C₃₋₈cycloalkyl-C₁₋₈alkyl; optionally substituted by one or more inert substituents; or are interlinked to form a ring of 4 to 7 ring atoms,
R₈ and R₉ are each C₁₋₈alkyl; C₃₋₈cycloalkyl; or C₃₋₈cycloalkyl-C₁₋₈alkyl; optionally substituted by one or more inert substituents; or are interlinked to form a ring of 4 to 7 ring atoms,
X is hydrogen or a hydrogen bridge forming group, and
m' and n' independently denotes an integer of zero to 5.

Specifically mentioned are the amines N,N,N',N'-tetramethyl-1,2-diaminoethane and 1,3-bis(dimethylamino)-2-propanol.

The amine may *e.g*. further be as described in WO 96/20199, such as an amine of formula 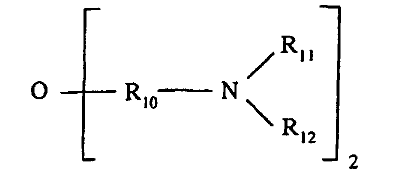 wherein R₁₀ is an alkylene group, optionally substituted by one or more inert substituents; and
R₁₁ and R₁₂ denote independently hydrogen or alkyl, optionally substituted by one or more inert substituents; or
R₁₁ and R₁₂ together with the nitrogen atom form a heterocyclic ring having 4 to 7 carbon atoms, optionally substituted by one or more inert substituents,
specifically bis(2-dimethylaminoethyl)ether.

The amine may *e.g*. further be as described in EP 729 961, such as an amine of formula 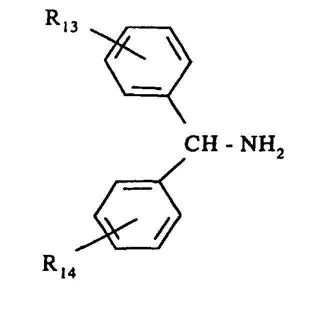 wherein
R₁₃ and R₁₄ independently represent a hydrogen atom or a pharmaceutically acceptable substituent.

The amine may *e.g.* further be as described in WO 94/21647, such as an amine of formula 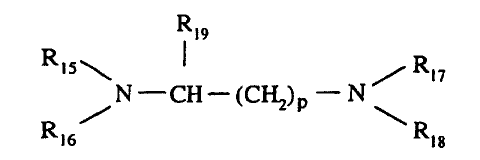 wherein
R₁₅, R₁₆, R₁₇, and R₁₈ denotes a hydrogen atom; a straight chain or a branched chain C₁₋₈alkyl group; an aralkyl group wherein the alkyl group is a methyl or ethyl group and the aryl group is a phenyl group, which is optionally substituted by an N-alkyl or N,N-dialkyl group wherein the alkyl groups are C₁₋₄ alkyl; or
R₁₅, R₁₆, R₁₇ and R₁₈ jointly independently denotes a cyclic alkylene ring having 3 to 6 methylene groups, one of these groups being optionally substituted by an oxygen or a sulphur atom or by an amino group; and
R₁₉ denotes a hydrogen atom or a methyl group; and
p denotes an integer from 1 to 3.
Particularly useful amines may be *e.g*. tert.butylamine, tert.octylamine, diisopropyl-ethylen-diamine, N,N,N',N'-tetramethyl-ethylendiamine, 1,3-bis(dimethylamino)-2-propanol and bis(2-dimethylaminoethyl)ether, such as such as tert.butylamine, tert.octylamine, diisopropyl-ethylen-diamine, N,N,N',N'-tetramethyl-ethylendiamine and bis(2-dimethylaminoethyl)ether, *e.g*. tert.butylamine, tert.octylamine.

In another aspect the present invention provides a process as defined above, characterized in that purification of clavulanic acid is effected *via* a salt of clavulanic acid with tert.butylamine, tert.octylamine, diisopropyl-ethylen-diamine, N,N,N',N'-tetramethyl-ethylendiamine, 1,3-bis(dimethylamino)-2-propanol or bis(2-dimethylaminoethyl)ether.

Purification under appropriate conditions is in more detail known from various publications, *e.g*. from references cited under step a), c) and d), and under step e) below, the content of which, including prior art citations therein, is incorporated herein by reference.

For salt formation the amine may be contacted with clavulanic acid in solution, in an organic solvent or solvent system. Conveniently the same solvent may be used which was used for extraction of clavulanic acid from the aqueous phase, for example of the optionally extracted and optionally pre-treated fermentation broth, *e.g*. ethyl acetate.
Prior to contacting the clavulanic acid containing organic solution with an amine the solution may be pre-concentrated, *e.g*. as described above.
Contacting clavulanic acid with the amine may be carried out by any appropriate method, for example the amine may be added to a solution of clavulanic acid in an organic solvent, for example by simple addition to the solution of clavulanic acid in a solvent, or, *e.g*. by mixing an amine into a stream of a solution of clavulanic acid in a solvent.
The desired salt of clavulanic acid with an amine may be isolated from the organic solvent as usual, for example by filtration or centrifugation, *e.g*. if the salt forms a solid, for examples if the salt crystallises.
A further solvent may be added to the mixture, *e.g.* a mixture containing clavulanic acid, a solvent and an amine, *e.g.* before, simultanously or after addition of the amine to a mixture of clavulanic acid and a solvent, that may cause precipitation of the amine salt of clavulanic acid.

The amine salt of clavulanic acid may be obtained in form of a solvate, for example of the acetone solvate. Recrystallisation of the amine salt of clavulanic acid may be effected. If the solvent is wholly or partly immiscible with water, the amine salt of clavulanic acid may also be extracted into water to form an aqueous solution of the salt, which may be very concentrated, as *e.g*. is described in WO 95/21173. Conditions such as reaction conditions, specific reaction conditions, reagents, amount ranges of reagents, concentration ranges, temperatures, etc. for purification and/or isolation of a salt of clavulanic acid, for example an amine salt, are known from, for example, references cited under points a), e) and c) and d) below, the content of which, including prior art citations therein, is incorporated herein by reference.

Conversion step e) may be carried out according to known methods.
In a process according to step e) clavulanic acid may be converted as such; or in form of a labile derivative thereof, for example a salt thereof, for example a lithium salt or a sodium salt thereof; or in form of an amine salt thereof.
A pharmaceutically acceptable salt of clavulanic acid may be, for example, a salt of clavulanic acid with pharmaceutically acceptable alkali or alkaline earth metals, preferably a potassium salt. Generally, clavulanic acid, or a salt thereof, for example an amine salt thereof, conveniently in solution, may be contacted with a cation source which is able to form a pharmaceutically acceptable salt of clavulanic acid. Suitable cation sources are described, for example in references cited above. A preferred cation source may be an alkali or earth alkali salt of a carboxylic acid, for example 2-ethylhexanoic acid, for example the potassium salt thereof, and *e.g*. an acetate, optionally in combination with acetic acid. In WO 97/18216, published 22.05.1997, a process for the production of a pharmaceutically acceptable salt of clavulanic acid by conversion of clavulanic acid into a pharmaceutically acceptable salt of clavulanic acid in n-butanol or iso-butanol (2-methyl-1-propanol) as a solvent is disclosed.
Conditions such as reaction conditions, specific reaction conditions, reagents, amount ranges of reagents, concentration ranges, temperatures, etc. for purification and/or isolation of a salt of clavulanic acid, for example an amine salt, are known from, for example, references cited under points a), c) d) and e), the content of which, including prior art citations therein, is incorporated herein by reference.

In one aspect a process according to the present invention may, for example be carried out as follows:
A fermentation broth containing clavulanic acid, having a a pH of *5.5* to *7.5*, *e.g*. obtained after fermentation with a clavulanic acid-producing micro-organism, may be extracted in a ratio by volume of 1 part fermentation broth to 0.5 to 3.0 parts, preferably 1.0 to 1.5 parts, of a solvent which is selected from the group consisting of an ester, a ketone, an ether, an alcohol or a mixture thereof, and which is able to form a liquid/liquid phase system with an aqueous fermentation broth. An appropriate solvent includes *e.g.* ethers, ketones, such as methyl isobutyl ketone, methyl ethyl ketone, diethyl ketone; esters, such as ethyl acetate, propyl acetate; and alcohols, such as n-butanol or i-butanol. Mixtures of individual solvents, *e.g*. as described above may be used. A solvent which subsequently may be used for extraction of the clavulanic acid from the aqueous, clavulanic acid containing mixture, *e.g*. after acidification, such as a ketone, ester and alcohol, e.g. ethyl acetate, methyl isobutyl ketone or diethyl ketone, *e.g.* ethyl acetate may preferably be used.
The clavulanic acid containing mixture may be adjusted, *e.g*. by use of an acidic agent, such as an acid to obtain a pH of 1.5 to 2.5, and the pH adjusted mixture may be extracted with a solvent which forms a liquid/liquid phase system with that mixture, selected from the group consisting of a ketone, an ester and an alcohol. Extraction may be also carried out simultaneously with acidification.
Clavulanic acid may be extracted into the organic phase. The organic phase containing clavulanic acid may *e.g*. be concentrated, purified, *e.g*. by use of activated carbon, *e.g*. as described above, mixed with a cosolvent, *e.g.* from the group acetone, methyl isobutyl ketone or diethyl ketone, *e.g*. acetone, and an amine salt of clavulanic acid may be precipitated. An amine salt of clavulanic acid may be isolated and converted into a pharmaceutically acceptable salt of clavulanic acid, *e.g*. the potassium salt, *e.g*. as described above.
Appropriate methods for such steps are described above and, *e.g*. described in references cited under point a) to e) above, the content of which is incorporated herein by reference including prior art citations therein.

In a further aspect a process according to the present invention may be carried out as follows: At least some of the solids in a fermentation broth containing clavulanic acid, *e.g.* obtained after fermentation with a clavulanic acid-producing micro-organism, may be removed, e.g. by filtration, such as precoat filtration and crossflow filtration. The filtrate obtained may be concentrated to a concentration of *e.g*. 10 g to 40 g clavulanic acid per kg, and may be treated with activated carbon, *e.g*. powdery, in a quantity of *e.g*. 0.5% to 5% of the whole mass. An aqueous concentrate of clavulanic acid obtained may be extracted at pH 5.5 to 7.5 in a ratio by volume of 1 part fermentation broth to 0.5 to 3.0 parts, preferably 1.0 to 1.5 parts, with an organic solvent which is able to form a liquid/liquid phase system with that concentrate, *e.g.* an extraction solvent as described above. Further processing of the clavulanic acid containing concentrate to obtain a pharmaceutically acceptable salt of clavulanic acid may take place according to a method as described above.

Appropriate methods for such steps are described above and, *e.g*. described in references cited under point a) to e) above, the content of which is incorporated herein by reference including prior art citations therein.

In general, filtration, extraction, removal of impurities and concentration may be carried out at any appropriate stage of the process according to the present invention, *e.g*. according to any method described or referred to above. Centrifugal separators or centrifugal decanters for any phase separation in a process according to the present invention may be used, e.g., particularly if the process is carried out on technical scale. Multi-staged countercurrent methods may be used. Appropriate flocculation agents or wetting agents may be added to the clavulanic acid containing mixture, *e.g.* as usual, *e.g.* in amounts of 0.001 to 1% w/w, if desired. A wetting agent includes *e.g*. Armogard D-5411®, cetyl trimethylammonium bromide, Dodigen®, Demulso® and a flocculation agent includes e.g Bozefloc®, Ferrocryl®, Locron®. For crossflow filtration, *e.g*. for separating off the biomass from the fermentation broth, there are a variety of commercial modular designs, *e.g*. plate, tubular or spiral moduli; the membrane materials may be *e.g.* polymeric or ceramic material. The separation ability of the membranes may vary from ca. 10 to 300 kD or 0.005 to 0.1 µm (UF range) up to ca. 0.1 to 2 µm (MF range). A normal crossflow pump speed may be 2 to 7m/s, preferably 4 to 6 m/s.

For thermal concentration of *e.g*. an aqueous, clavulanic acid containing liquid, special vacuum evaporators may be used, *e.g.* operating at very low pressures and with short retention time. Appropriate special vacuum evaporators include thin-layer evaporators. An alternative may be a membrane process, such as reversed osmosis or nanofiltration. Appropriate membrane materials including *e.g.* polymers and ceramics and appropriate modular designs including *e.g.* spirals, tubes, plates are known. An additional purification of *e.g.* a concentrate *e.g.* obtained by reversed osmosis or nanofiltration of high molecular compounds may take place effectively through, *e.g*. narrow ultrafiltration membranes, which are available commercially *e.g*. as spiral moduli having a separation ability of 1 to 3 kD.

Impurities may be removed at any stage of the process of the present invention. Appropriate methods for removing impurities are described above and, *e.g*. described in references cited under point a) to e) above, the content of which is incorporated herein by reference including prior art citations therein.

In another aspect the present invention provides a process for the production of clavulanic acid and its alkali salts by extraction of clavulanic acid from the culture broth obtained by microbial fermentation, reaction with an amine to form an amine salt of clavulanic acid and reaction to form clavulanic acid alkali salts, characterised in that
a) the entire unfiltered fermentation broth obtained after fermentation with a microorganism which produces clavulanic acid is extracted, at a native pH (*5.5* - *7.5*), with a solvent which is immiscible with water, selected from ethers, ketones, esters or alcohols to provide an aqueous fermentation broth containing clavulanic acid; or
b) the fermentation broth obtained after fermentation with a micro-organism which produces clavulanic acid is filtrated by use of usual filtration processes; after optional pre-treatment by addition of flocculation agents; the culture filtrate is concentrated and optionally purified, and afterwards extracted at a pH of 5.5 - 7.5 with a solvent which is immiscible with water, selected from ethers, ketones, esters or alcohols, to provide an aqueous fermentation broth containing clavulanic acid,
clavulanic acid is subsequently extracted from the resulting aqueous fermentation broth containing clavulanic acid obtained after the extraction at a pH of 5.5-7.5, in several stages at an acidic pH of 1.5 to 2.5 into a solvent which is immiscible with water selected from ketones, esters or alcohols, and reacted by known methods to obtain an amine salt of clavulanic acid, and an amine salt obtained is converted by known methods into a pharmaceutically acceptable salt of clavulanic acid.

A solvent which is immiscible with water is regarded herein to be a solvent which is able to form a liquid/liquid phase system in contact with an aqueous mixture, *e.g.* an aqueous fermentation broth.

Owing to its inhibiting activity on β-lactamases, clavulanic acid, a compound of formula I, *i.e*. (2R,5R,Z)-3-(2-hydroxyethylidene)-7-oxo-4-oxa-1-aza-bicyclo-[3,2,0]-heptane-2-carboxylic acid, *e.g*. in form of a salt, *e.g.* in form of a potassium salt is useful as an additive for β-lactam antibiotic formulations, *e.g.* in combination with amoxicillin, *e.g*. in form of a trihydrate; and a process according to the present invention is useful for the provision of clavulanic acid.
The present invention may be viewed as an alternative to other commercially useful processes for producing clavulanic acid and/or pharmaceutically acceptable salts thereof. It may be viewed as an unobvious improvement to processes as described in processes of prior art, including processes of references cited above under points a) to e).

The following examples illustrate the invention without limiting its scope. All temperatures are given in degrees Celsius.

### Example 1

105 kg of a fermentation broth containing clavulanic acid are treated using crossflowultra/diafiltration for the removal of biomass. 200 kg of a permeate are obtained which is concentrated to obtain a clavulanic acid content of about 15 g/kg by use of reversed osmosis under cooling. 34 kg of the concentrate obtained are extracted at neutral pH, whilst cooling, with three times the amount of ethyl acetate. The extracted concentrate obtained is extracted at pH 2 under cooling, in several stages, with ethyl acetate. A clavulanic acid extraction yield of 80%, determined by HPLC is obtained. The clavulanic acid containg extract is concentrated by evaporation *in vacuo* to give a concentrate containing ca. 8 g clavulanic acid per kg and the concentrate obtained is dried over anhydrous sodium sulphate. Activated carbon is added to the mixture containing clavulanic acid, ethyl acetate and sodium sulphate, and the mixture obtained is clear-filtrated. The filtrate is evaporated to give a concentrate containing about 30 g clavulanic acid per kg and the concentrate is diluted with acetone in a ratio of about 1:1. A 15% solution of tert.butylamine in acetone at about 20° is added until a pH of ca. 6.1 is adjusted. Crystallisation occurs and the precipitate is isolated, washed with acetone and dried. An acetone solvate of a tert.butylamine salt of clavulanic acid is obtained. Content of clavulanic acid tert.butylamine salt (HPLC): 95% on a solvent-free basis

### Example 2

900 kg of a clavulanic acid containing fermentation broth (pH 7.0) are filtrated by crossflow ultrafiltration. De-ionised water is added and the mixture is diafiltrated. 2700 kg of a filtrate containing clavulanic acid are obtained. 1200 kg of a filtrate obtained are concentrated by use of reversed osmosis/nanofiltration at 4° to give a concentrate containing 9 g of clavulanic acid per kg. The concentrate is ultrafiltrated through a membrane (2 to 3 kD). 62 kg of the permeate obtained are extracted at neutral pH with three times the quantity of ethyl acetate. The aqueous solution is extracted continuously with four times the amount of ethyl acetate under cooling, at pH 2.0, using a two-stage counter-current extraction apparatus (2 centrifugal separators), 320 kg of ethyl acetate are concentrated by evaporation in an agitator vessel evaporator to obtain a concentrate containing ca. 80 g clavulanic acid per kg. 1.9 kg of the concentrate obtained are treated with 0.3 times the quantity of activated carbon based on the clavulanic acid mass and the mixture is clear-filtrated. The carbon is washed with ethyl acetate until the concentration in the filtrate is 30 g clavulanic acid per kg. The same quantity of acetone is added to the filtrate. 1.2 equivalents of a 25% solution of tert.octylamine in acetone at 20° are added and a pH of 6.3 is adjusted. After cooling to 4°, a tert.octylamine salt of clavulanic acid is obtained in crystalline form, isolated, washed with acetone and dried. Content of clavulanic add tert. octylamine salt (HPLC): 98% (absolutely dry).

### Example 3

1000 kg of a fermentation broth (pH 7.0) containing clavulanic acid arc filtrated to obtain 750 kg clavulanic acid containing concentrate by use of crossflow ultrafiltration. The concentrate obtained is diafiltered in the presence of 2200 kg of de-ionised water. About 1000 kg of a filtrate obtained are concentrated by use of nano-filtration to give a concentrate containing 6 to 10 g, clavulanic acid per kg. About 140 kg of the concentrate obtained are extracted with 340 kg of ethyl acetate at around neutral pH. The extracted concentrate is continuously extracted with 700 kg of ethyl acetate under cooling at pH 2.0, using a two-stage countercurrent extraction device. The ethyl acetate extract is evaporated *in vacuo* to give a concentrate containing 37 g clavulanic acid per kg. The concentrate obtained is treated with 0.5 times the quantity of activated carbon (Noritc CG1®), based on the clavulanic acid mass, and the mixture obtained is clear-filtrated The filtrate obtained is diluted with acetone (ca. 1:1, v/v). A 25% solution of tert.octylamine in acetone is slowly added at 20° until a pH of 6.0 is adjusted. Ca. 1.1 equivalents of tert.octylamine in respect with calvulanic acid are used. The mixture is cooled to 4°. The tert.octylamine salt of clavulanic acid crystallises, is isolated and dried. The tert.octylamine salt of clavulanate is obtained in a yield of 65% and a purity of 98% (absolutely dry) determined by HPLC.

### Example 4

A clavulanic acid containing fermentation broth is filtrated by use of a crossflow-ultrafiltration device and biomass is removed. The filtrate is concentrated by use of reversed osmosis to obtain a concentrate with a clavulanic acid content of ca. 10 g per liter. 500 ml of the concentrate obtained are treated with 500 ml of ethyl acetate at a pH of 5.5 and the mixture is stirred for ca. 10 minutes. The mixture is filtrated and the phases are separated. The aqueous phase is treated with 500 ml of ethyl acetate and 20% aqueous sulphuric acid is added under stirring at 5° until a pH of 2.0 is adjusted. The phases are separated by use of centrifugation and the aqueous phase is again extracted with ethyl acetate. The ethyl acetate phases obtained are combined and concentrated by evaporation to a volume of ca. 150 ml. Active carbon is added, the suspension obtained is stirred and activ carbon is filtrated off. 120 ml acetone are added to the filtrate. 1.1 equivalents of tert.octylamine in respect with clavulanic acid (HPLC determination) are added dropwise to the mixture. A precipitate occurs, the mixture is stirred and the precipitate is filtrated off, washed with acetone and dried *in vacuo*. A tert.octylamine salt of clavulanic acid is obtained. Content of clavulanic acid tert. octylamine salt (HPLC): 98.4% (related with dry compound).

### Comparisan Example

A filtrated, concentrated fermentation liquid obtained as described in Example 4 with a clavulanic acid content of ca. 10 g per liter is treated with 500 ml of ethyl acetate and 20% aqueous sulphuric acid is added under stirring at 5° until a pH of 2.0 is adjusted. Further treatment of the mixture obtained is as described in Example 4 for the mixture obtained after addition of sulphuric acid. A tert.octylamine salt of clavulanic acid is obtained.
Content of clavulanic acid tert. octylamine salt (HPLC): 95.2% (related with dry compound).

### Example 5

720 kg of a fermentation broth containing clavulanic acid are extracted at neutral pH (6.8) with 500 1 of ethyl acetate. The aqueous phase is cooled to 4° and a pH of 2 is adjusted by use of sulphuric acid. The acidified aqueous phase is extracted with ethyl acetate in several steps. The ethyl acetate phase is concentrated to obtain a concentrate containing ca. 40 g of clavulanic acid per kg. The concentrate obtained is treated with ca. 1.0 times the quantity of activated carbon based on the clavulanic acid mass and the mixture is filtrated. The filtrate obtained is diluted with acetone (ca. 1:1 v/v) and a solution of tert.octylamine (25% in acetone) is added until a pH of ca. 6.0 is adjusted. A suspension is obtained which is cooled and the precipitated clavulanic acid tert. octylamine salt is isolated and dried.
Yield of clavulanic acid tert. octylamine salt: 60%
Purity of clavulanic acid tert. octylamine salt: 99.1% (related to dry compound, HPLC)

In each of the above Examples the amine specified may be replaced by any amine which is indicated above to be useful in the isolation of clavulanic acid. Equivalent results may be obtained, preferably by use of tert.butylamine, tert.octylamine, diisopropyl-ethylen-diamine, N,N,N',N'-tetramethyl-ethylendiamine, 1,3-bis(dimethylamino)-2-propanol and bis(2-dimethylaminoethyl)ether, such as tert.butylamine, tert.octylamine, diisopropyl-ethylen-diamine, N,N,N',N'-tetramethyl-ethylendiamine and bis(2-dimethylaminoethyl)ether.

## Claims

1. A process for the production of clavulanic acid and pharmaceutically acceptable salts thereof comprising
a) fermentation of a micro-organism which is capable of producing clavulanic acid to provide a fermentation broth containing clavulanic acid,
b) extraction of the fermentation broth containing clavulanic acid with an organic solvent that is able to form a liquid/liquid phase system in contact with an aqueous fermentation broth, selected from the group consisting of an ether, a ketone, an ester, an alcohol and a mixture thereof at a pH of 5.5-7.5 to provide an extracted aqueous fermentation broth whereby the clavulanic acid remains substantially in the aqueous fermentation broth,
c) extraction of clavulanic acid from said extracted aqueous fermentation broth at a pH of 1.5 to 2.5 with a solvent in which clavulanic acid is soluble under the extraction conditions and which is able to form a liquid/liquid phase system in contact with the aqueous fermentation broth, and which is selected from the group consisting of an ester, a ketone, an alcohol and a mixture thererof,
d) purification of the extracted clavulanic acid obtained in step c) or a salt thereof, and
e) conversion of the purified clavulanic acid obtained in step d) or a salt thereof into a pharmaceutically acceptable salt of clavulanic acid.

2. A process according to claim 1 wherein at least part of the solids suspended in the fermentation broth containing clavulanic acid of step a) are removed prior to extraction.

3. A process according to one of the preceding claims, wherein in that the fermentation broth containing clavulanic acid of step a) is concentrated prior to extraction.

4. A process according to one of the preceding claims, wherein the extraction of clavulanic acid of step c) is effected using the same solvent that was used for the extraction of the fermentation broth containing clavulanic acid of step b).

5. A process according to claim 4, wherein the solution of clavulanic acid in said second solvent is concentrated prior to purification step d).

6. A process according to any one of the preceding claims wherein the purification of the isolated clavulanic acid of step d) is effected by a salt of clavulanic acid with tert.butylamine, tert.octylamine, diisopropyl-ethylen-diamine, N,N,N',N'-tetramethylethylendiamine, 1,3-bis(dimethylamino)-2-propanol or bis(2-dimethylaminoethyl)ether.

7. A process according to any one of the preceding claims wherein the pharmaceutically acceptable salt of clavulanic acid is a potassium salt of clavulanic acid.

8. A process for the production of clavulanic acid and its alkali salts according to claim 1 comprising the steps of
A) isolation of clavulanic acid by extraction from a culture broth obtained by microbial fermentation,
B) reaction of said clavulanic acid with an amine to form an amine salt of clavulanic acid, and
C) conversion of said amine salt of clavulanic acid into a clavulanic acid alkali salt, **characterised in that**
i) a) the culture broth obtained after fermentation with a microorganism which produces
clavulanic acid, is extracted at a native pH of 5.5 - 7.5 with a solvent which is immiscible with water, selected from ethers, ketones, esters or alcohols, to provide an aqueous containing fermentation broth containing clavulanic acid or
b) the culture broth obtained after fermentation with a microorganism which produces
clavulanic acid is, after optional pre-treatment by addition of flocculation agents, filtrated by known methods to provide a filtrate, said filtrate is concentrated, optionally purified, and subsequently extracted at a pH of 5.5- 7.5 with a solvent which is immiscible with water, selected from ethers, ketones, esters or alcohol, to provide an aqueous containing fermentation broth containing clavulanic acid,
ii) clavulanic acid is extracted from the resulting aqueous fermentation broth
containing clavulanic acid obtained after the extraction in step (i), in several stages at an acidic pH of 1.5 to 2.5 into a solvent which is immiscible with water, selected from ketones, esters or alcohols, and converted by known methods into an amine salt of clavulanic acid, and
iii) said amine salt of clavulanic acid is converted by known methods into a pharmaceutically acceptable salt of clavulanic acid.

## Patentansprüche

1. Verfahren zur Herstellung von Clavulansäure und pharmazeutisch annehmbarer Salze davon, umfassend
a) die Fermentation eines Mikroorganismus, der fähig ist, Clavulansäure zu erzeugen, so dass eine Clavulansäure-haltige Fermentationsbrühe entsteht,
b) die Extraktion der Clavulansäure-haltigen Fermentationsbrühe mit einem organischen Lösungsmittel, das fähig ist, im Kontakt mit einer wässrigen Fermentationsbrühe ein Flüssigkeits/Flüssigphasen-System zu bilden, ausgewählt aus der Gruppe aus einem Ether, einem Keton, einem Ester, einem Alkohol und einem Gemisch davon, bei einem pH-Wert von 5,5 bis 7,5, so dass eine extrahierte, wässrige Fermentationsbrühe entsteht, wobei die Clavulansäure im wesentlichen in der wässrigen Fermentationsbrühe zurückbleibt,
c) die Extraktion von Clavulansäure aus der extrahierten, wässrigen Fermentationsbrühe bei einem pH-Wert von 1,5 bis 2,5 mit einem Lösungsmittel, in dem Clavulansäure unter den Extraktionsbedingungen löslich ist und welches fähig ist, im Kontakt mit der wässrigen Fermentationsbrühe ein Flüssigkeits/Flüssigphasen-System zu bilden, und welches ausgewählt ist aus der Gruppe aus einem Ester, einem Keton, einem Alkohol und einem Gemisch davon,
d) die Reinigung der in Schritt c) gewonnenen, extrahierten Clavulansäure oder eines Salzes davon und
e) die Umwandlung der in Schritt d) gewonnenen, gereinigten Clavulansäure oder eines Salzes davon in ein pharmazeutisch annehmbares Clavulansäuresalz.

2. Verfahren nach Anspruch 1, wobei wenigstens ein Teil der in der Clavulansäure-haltigen Fermentationsbrühe aus Schritt a) suspendierten Feststoffe vor der Extraktion entfernt werden.

3. Verfahren nach einem der vorstehenden Ansprüche, wobei die Clavulansäure-haltige Fermentationsbrühe aus Schritt a) vor der Extraktion aufkonzentriert wird.

4. Verfahren nach einem der vorstehenden Ansprüche, worin die Extraktion von Clavulansäure aus Schritt c) mit Hilfe des gleichen Lösungsmittels durchgeführt wird, das für die Extraktion der Clavulansäure-haltigen Fermentationsbrühe aus Schritt b) verwendet wurde.

5. Verfahren nach Anspruch 4, wobei die Clavulansäurelösung in dem zweiten Lösungsmittel vor dem Reinigungsschritt d) aufkonzentriert wird.

6. Verfahren nach einem der vorstehenden Ansprüche, wobei die Reinigung der isolierten Clavulansäure aus Schritt d) durch ein Salz von Clavulansäure mit tert-Butylamin, tert-Octylamin, Diisopropyl-ethylendiamin, N,N,N',N'-Tetramethyl-ethylendiamin, 1,3-Bis(dimethylamino)-2-propanol oder Bis(2-dimethylaminoethyl)ether durchgeführt wird.

7. Verfahren nach einem der vorstehenden Ansprüche, wobei das pharmazeutisch annehmbare Clavulansäuresalz das Kaliumsalz von Clavulansäure ist.

8. Verfahren zur Herstellung von Clavulansäure und seiner alkalischen Salze gemäß Anspruch 1, umfassend die Schritte
A) Isolierung von Clavulansäure durch Extraktion einer Kulturnährlösung, die mittels mikrobieller Fermentation gewonnen wurde,
B) Umsetzen der Clavulansäure mit einem Amin, so dass ein Aminsalz von Clavulansäure entsteht, und
C) Umwandeln des Aminsalzes von Clavulansäure in ein Clavulansäurealkalisalz, **dadurch gekennzeichnet, dass**
i) a) die durch Fermentation mit einem Clavulansäure-produzierenden Mikroorganismus gewonnene Kulturnährlösung bei einem nativen pH-Wert von 5,5 bis 7,5 mit einem Lösungsmittel extrahiert wird, das unmischbar mit Wasser ist und aus Ethern, Ketonen, Estern oder Alkoholen ausgewählt ist, so dass eine Clavulansäure-haltige wässrige Fermentationsbrühe bereitgestellt wird, oder
b) die durch Fermentation mit einem Clavulansäure-produzierenden Mikroorganismus gewonnene Kulturnährlösung, gegebenenfalls nach einer Vorbehandlung durch Zugeben von Flockungsmitteln, durch bekannte Verfahren filtriert wird, so dass ein Filtrat entsteht, das Filtrat aufkonzentriert, gegebenenfalls gereinigt und anschließend bei einem pH-Wert von 5,5 bis 7,5 mit einem Lösungsmittel extrahiert wird, welches unmischbar mit Wasser ist und ausgewählt ist aus Ethern, Ketonen, Estern oder Alkoholen, so dass eine wässrige, Clavulansäure-haltige Fermentationsbrühe ensteht,
ii) Clavulansäure aus der nach der Extraktion in Schritt i) gewonnenen wässrigen, Clavulansäure-haltigen Fermentationsbrühe in mehreren Stufen bei einem sauren pH-Wert von 1,5 bis 2,5 in ein Lösungsmittel extrahiert wird, welches mit Wasser unmischbar ist und ausgewählt ist aus Ketonen, Estern oder Alkoholen, und durch bekannte Verfahren in ein Aminsalz von Clavulansäure umgewandelt wird, und
iii) das Aminsalz von Calvulansäure durch bekannte Verfahren in ein pharmazeutisch annehmbares Clavulansäuresalz umgewandelt wird.

## Revendications

1. Procédé pour la production d'acide clavulanique et de ses sels pharmaceutiquement acceptables, comprenant
a) la fermentation d'un micro-organisme qui est capable de produire de l'acide clavulanique pour fournir un bouillon de fermentation contenant l'acide clavulanique,
b) l'extraction du bouillon de fermentation contenant l'acide clavulanique avec un solvant organique capable de former un système de phase liquide/liquide au contact d'un bouillon de fermentation aqueux et choisi dans le groupe constitué par un éther, une cétone, un ester, un alcool et un de leurs mélanges, à un pH de 5,5-7,5 pour fournir un extrait de bouillon de fermentation aqueux pour lequel l'acide clavulanique reste substantiellement dans le bouillon de fermentation aqueux,
c) l'extraction de l'acide clavulanique dudit extrait de bouillon de fermentation aqueux, à un pH de 1,5 à 2,5, avec un solvant dans lequel l'acide clavulanique est soluble dans les conditions d'extraction et qui est capable de former un système de phase liquide/liquide au contact du bouillon de fermentation aqueux, et qui est choisi dans le groupe constitué par un ester, une cétone, un alcool et un de leurs mélanges,
d) la purification de l'acide clavulanique extrait, obtenu dans l'étape c) ou d'un sel de celui-ci, et
e) la conversion de l'acide clavulanique purifié, obtenu dans l'étape d) ou d'un sel de celui-ci en un sel pharmaceutiquement acceptable d'acide clavulanique.

2. Procédé selon la revendication 1, dans lequel au moins une partie des solides en suspension dans le bouillon de fermentation contenant l'acide clavulanique de l'étape a) sont éliminés avant l'extraction.

3. Procédé selon l'une quelconque des revendications précédentes, dans lequel le bouillon de fermentation contenant l'acide clavulanique de l'étape a) est concentré avant l'extraction.

4. Procédé selon l'une quelconque des revendications précédentes, dans lequel l'extraction de l'acide clavulanique de l'étape c) s'effectue en utilisant le même solvant que celui utilisé pour l'extraction du bouillon de fermentation contenant l'acide clavulanique de l'étape b).

5. Procédé selon la revendication 4, dans lequel la solution d'acide clavulanique dans ledit second solvant est concentrée avant l'étape de purification d).

6. Procédé selon l'une quelconque des revendications précédentes, dans lequel la purification de l'acide clavulanique isolé de l'étape d) s'effectue grâce à un sel d'acide clavulanique avec la tert-butylamine, la tert-octylamine, la diisopropyléthylènediamine, la N,N,N',N'-tétraméthyléthylènediamine, le 1,3-bis(diméthylamino)-2-propanol ou le bis(2-diméthylaminoéthyl)éther.

7. Procédé selon l'une quelconque des revendications précédentes, dans lequel le sel pharmaceutiquement acceptable d'acide clavulanique est un sel de potassium d'acide clavulanique.

8. Procédé pour la production d'acide clavulanique et de ses sels alcalins selon la revendication 1, comprenant les étapes de
A) isolement de l'acide clavulanique par extraction à partir d'un bouillon de culture obtenu par fermentation microbienne,
B) réaction dudit acide clavulanique avec une amine pour former un sel d'amine d'acide clavulanique, et
C) conversion dudit sel d'amine d'acide clavulanique en un sel alcalin d'acide clavulanique,
**caractérisé en ce que**
i) a) le bouillon de culture obtenu après fermentation avec un micro-organisme qui produit de l'acide clavulanique est extrait à un pH natif de 5,5-7,5 avec un solvant non miscible à l'eau choisi parmi les éthers, les cétones, les esters ou les alcools, pour fournir un bouillon de fermentation aqueux contenant l'acide clavulanique ou
b) le bouillon de culture obtenu après fermentation avec un micro-organisme qui produit de l'acide clavulanique est filtré par des procédés connus après un prétraitement éventuel par addition d'agents de floculation pour fournir un filtrat, ledit filtrat est concentré, purifié éventuellement et ensuite extrait à un pH de 5,5-7,5 avec un solvant non miscible à l'eau, choisi parmi les éthers, les cétones, les esters ou les alcools, pour fournir un bouillon de fermentation aqueux contenant l'acide clavulanique,
ii) l'acide clavulanique est extrait à partir du bouillon de fermentation aqueux résultant contenant l'acide clavulanique obtenu après l'extraction de l'étape (i), en plusieurs étapes, à un pH acide de 1,5 à 2,5 dans un solvant non miscible à l'eau choisi parmi les cétones, les esters ou les alcools, et converti grâce à des procédés connus en un sel d'amine d'acide clavulanique, et
iii) ledit sel d'amine d'acide clavulanique est converti grâce à des procédés connus en un sel pharmaceutiquement acceptable d'acide clavulanique.
